# EUROPEAN PATENT SPECIFICATION

(11) **EP 1 732 569 B1**
(45) Date of publication and mention of the grant of the patent: **14.10.2009**
(21) Application number: 05706024.6
(22) Date of filing: 21.01.2005
(51) Int. Cl.: A61K 31/675, A61K 45/06, A61P 35/00, A61P 1/16, A61P 31/12

(54) **USE OF ADEFOVIR OR TENOFOVIR FOR INHIBITING MMTV-LIKE VIRUSES INVOLVED IN BREAST CANCER AND PRIMARY BILIARY CIRRHOSIS**
VERWENDUNG VON ADEFOVIR ODER TENOFOVIR ZUR HEMMUNG VON MMTV-ARTIGEN VIREN IM ZUSAMMENHANG MIT BRUSTKREBS UND PRIMÄRER BILIÄRER ZIRRHOSE
UTILISATION D'ADEFOVIR OU DE TENOFOVIR POUR INHIBER LES VIRUS SEMBLABLES A MMTV IMPLIQUES DANS LE CANCER DU SEIN ET LA CIRRHOSE BILIAIRE PRIMAIRE

(30) Priority: 21.01.2004 US 538066 P
(43) Date of publication of application: 20.12.2006
(73) Proprietor: GILEAD SCIENCES, INC., Foster City, California 94404 (US)
(72) Inventor: CIHLAR, Tomas, Foster City, CA 94404 (US); DOUGLAS, Janet, L., San Francisco, CA 94110 (US); GIBBS, Craig, S., Palo Alto, CA 94301 (US)
(74) Representative: Reitstötter - Kinzebach
(86) International application number: PCT/US2005/002043
(87) International publication number: WO 2005/072748

(56) References cited:
- WO-A-02/08241
- US-A1- 2003 229 225
- US-B1- 6 468 737
- MASON ANDREW ET AL: "Primary biliary cirrhosis: new thoughts on pathophysiology and treatment." CURRENT GASTROENTEROLOGY REPORTS. FEB 2002, vol. 4, no. 1, February 2002 (2002-02), pages 45-51, XP008046866 ISSN: 1522-8037 cited in the application
- KEEFFE E B ET AL: "Advances in liver disease. Highlights from the 54th Annual Meeting of the American Association for the Study of Liver Diseases, October 24-28, 2003" REVIEWS IN GASTROENTEROLOGICAL DISORDERS 2004 UNITED STATES, vol. 4, no. 1, 2004, pages 36-42, XP008046946 ISSN: 1533-001X
- WALSH K M ET AL: "Successful treatment with adefovir dipivoxil in a patient with fibrosing cholestatic hepatitis and lamivudine resistant hepatitis B virus." GUT, (2001 SEP) 49 (3) 436-40. JOURNAL CODE: 2985108R. ISSN: 0017-5749., September 2001 (2001-09), XP008046882
- ANDREI G ET AL: "Antiproliferative effects of acyclic nucleoside phosphonates on human papillomavirus (HPV)-harboring cell lines compared with HPV-negative cell lines" ONCOLOGY RESEARCH, vol. 10, no. 10, 1998, pages 523-531, XP008046881 ISSN: 0965-0407
- KAST R E (REPRINT): "Tenofovir , COX inhibitors and zileuton during cancer immunotherapies: up-regulated TNF-alpha increases antigen driven lymphocyte proliferation" MOLECULAR IMMUNOLOGY, (SEP 2003) VOL. 40, NO. 5, PP. 297-303. PUBLISHER: PERGAMON-ELSEVIER SCIENCE LTD, THE BOULEVARD, LANGFORD LANE, KIDLINGTON, OXFORD OX5 1GB, ENGLAND. ISSN: 0161-5890., September 2003 (2003-09), XP008046864
- XU LIZHE ET AL: "Does a betaretrovirus infection trigger primary biliary cirrhosis?" PROCEEDINGS OF THE NATIONAL ACADEMY OF SCIENCES OF THE UNITED STATES OF AMERICA, vol. 100, no. 14, 8 July 2003 (2003-07-08), pages 8454-8459, XP008046857 ISSN: 0027-8424 cited in the application
- O'NEIL M. ET AL.: "The Merck Index Thirteenth Edition" 2001, MERCK & CO, INC. , WHITEHOUSE STATION, N.J. , XP002328456 page 1631, column 2 - page 1362, column 1
- O'NEIL M. ET AL.: "The Merck Index Thirteenth Edition" 2001, MERCK & CO. INC. , WHITEHOUSE STATION, N.J. , XP002328457 page 29, column 1

## Description

### Cross-reference to Related Applications

This application claims priority from U.S. provisional patent application No. 60/538,066, filed January 21,2004.

### Background of the Invention

Human primary biliary cirrhosis (PBC) is an autoimmune disorder that is characterized by a progressive destruction of the small intrahepatic bile ducts, which ultimately leads to liver failure and the need for a liver transplant (Neuberger, J., Lancet 350:875-879, 1997). The hallmark of this disease is the presence of auto-antibodies to the mitochondrial pyruvate dehydrogenase complex (PDC-E2) (Nishio, A., et al., Hepatology 25:1085-1089, 1997). This complex is normally expressed in the mitochondrial inner membrane, but in most PBC patients is aberrantly localized to the cell surface of biliary epithelial cells and macrophages in non-hepatic lymph nodes (Joplin, R. and Gershwin, M.E., Seminars in Liver Disease 17:97-103, 1997). This aberrant expression of PDC-E2 may play a role in the pathogenesis of PBC. Currently, the only approved treatment for PBC is the bile acid, ursodeoxycholic acid (URSO). For some patients, URSO improves the liver biochemical markers and slows disease progression, however more than 65% of patients still develop progressive disease and require liver transplants (Lee, Y. and Kaplan M., Current Gastroenterology Reports 1:38-41, 1999). A treatment that inhibits the cause of the disease, not just the effects, has the potential to be much more beneficial than URSO alone. It has been hypothesized that an infectious agent or environmental trigger may be responsible for the development of PBC. Recently, a new exogenous β-retrovirus was cloned from PBC patients and can be detected in approximately 75% of PBC patients by RT-PCR (Xu, L., et al., PNAS 100:8454-8459, 2003). The new viral sequences are 91-99% homologous to human proviral sequences from breast cancer tissue and multiple strains of mouse mammary tumor virus (MMTV). Because of the high homology between the newly identified human retrovirus and MMTV, it is possible that this virus is a variant of MMTV that has crossed species and is not actually a new human virus. Electron microscopy studies revealed β-retrovirus-like particles *in vivo* and *in vitro* in liver cell samples from PBC patients. In addition, the PBC phenotype as measured by the aberrant localization of PDC- E2 could be induced in normal primary biliary epithelial cells (BEC) by incubation with conditioned media from PBC patient lymph nodes. These results suggested that the new human β-retrovirus might be the etiological agent of PBC. Therefore, several pilot clinical studies were undertaken to determine the-efficacy of two standard HIV anti-retroviral therapies in the treatment of PBC patients. The reverse transcriptase (RT) inhibitors, lamivudine (3TC, 150 mg), daily, and Combivir® (3TC/AZT, 150mg/ 300mg) twice daily, were given to PBC patients for 1 year. After 6 months the patients in the lamivudine arm showed no significant improvement in several liver function parameters. However, significant improvement in these liver function markers was evident in the Combivir treatment arm. Analysis of liver biopsies also revealed improved liver histology only in the Combivir treated patients (Mason, A., et al., Abstracts 54th Annual Meeting of AASLD, 2003). Interestingly, the levels of anti-mitochondrial antibodies in patients treated with both drug regimens were significantly reduced (Mason, A. and Nair, S., Current Gastroenterology Reports 4:45-51, 2002). AZT triphosphate has been shown to directly inhibit the MMTV RT enzyme as efficiently as the HIV RT enzyme (Wu, J., et al., J. Biol. Chem. 268:9980-9985, 1993). This suggests that the apparent clinical benefit of Combivir in PBC patients might be a consequence of the direct antiviral effect of AZT. In addition to PBC, viral envelope sequences with a high homology to the corresponding sequences of MMTV have also been identified in 38% of sampled human breast tumors (Wang, Y., et.al., Cancer Research 55:5173-5179, 1995). The entire proviral sequence has been cloned and is approximately 95% homologous to MMTV (Liu, B., et al., Cancer Research 61:1754-1759, 2001). The effect of anti-retrovirals on the development or progression of breast cancer has not been evaluated.

While these data are not direct evidence that the newly identified β-retrovirus is the causative agent for PBC and/or breast cancer it warrants further investigation into the potential therapeutic benefits of HIV RT inhibitors for the treatment of both PBC and breast cancer. The observation that lamivudine appeared less efficacious than Combivir in the PBC studies also suggests that a variety of RT inhibitors should be evaluated to identify the most effective one.

Mason et al., in U.S. Patent No. 6,468,737, issued October 22, 2002, reported the discovery, identification, and characterization of novel nucleic acid molecules that are associated with PBC. These nucleotide sequences are retroviral in origin and are indicative of a PBC retrovirus which bears a strong correlation with ABC. This is believed to be the first evidence to suggest that PBC patient's tissue may harbor a transmissible agent. Mason et al. assessed the efficacy and biologic response of a reverse transcriptase inhibitor in PBC patients using lamivudine 150 mg per day for one year. Of the 10 patients treated none had a complete biochemical response to treatment, but 8 patients had a reduction in their serum AMA levels and 2 had no change. Their studies suggest that the susceptibility of the PBC retrovirus to reverse transcriptase inhibition is unpredictable.

The acyclic nucleoside phosphonates, 2-R-(phosphonomethoxy)propyl adenine (PMPA, tenofovir) and 2-(phosphonomethoxy)ethyl adenine (PMEA, adefovir) are RT inhibitors with potent antiviral activity that have been approved by the FDA for the treatment of HIV and HBV infection, respectively. The approved versions of these drugs, Hepsera® (adefovir) and Viread® (tenofovir) are available worldwide from Gilead Sciences, Inc. and its commercial partners. Inside the target cells, cellular kinases convert these phosphonates into their diphosphate analogs, (PMPApp and PMEApp), which directly inhibit HIV RT (Holy, A., in Recent Advances in Nucleosides: Chemistry and Chemotherapy, C. K. Chu (ed.), 167-238, 2002).

Reviews in Gastroenterological Disorders 2004 United States, vol. 4, no. 1, 2004, pages 36 to 42 concerns advances in liver diseases. It is disclosed that primary biliary cirrhosis may be treated using Ursoviol and Methotrexat whereas Adefovir, Adefovir Dipivoxil and Tenofovir may be alternatives for the treatment of hepatitis B. Oncology, research, vol. 10, pp. 523-531, 1998, is concerned with the anti-proliferative effects of acyclic nucleoside phosphonates on human papilloma virus (HPV)-harbouring cell lines. Molecular immunology 40 (2003) 297-303 discloses the use of Tenofovir in cancer immunotherapy.

### Summary of the Invention

The active metabolites of adefovir and tenofovir (PMEApp and PMPApp) are active against the MMTV RT. They are 25-fold more potent than 3TCppp, suggesting that tenofovir and adefovir may be effective at inhibiting the MMTV-like retroviruses, which may be the etiological agents involved in PBC and breast cancer.

One aspect of the invention relates to the use of adefovir or tenofovir for preparing a pharmaceutical formulation for treating mammalian breast cancer wherein MMTV-like retroviruses are the etiological agents or human primary biliary cirrhosis wherein MMTV-like retroviruses are the etiological agents by inhibiting the activity of MMTV-like retrovirus reverse transcriptase.

In another aspect, the invention relates to the use of adefovir or tenofovir for preparing a pharmaceutical formulation for treating human primary biliary cirrhosis wherein MMTV-like retroviruses are the etiological agents or mammalian breast cancer wherein MMTV-like retroviruses are the etiological agents by inhibiting the replication of MMTV-like retroviruses.

In another aspect, the invention relates to the use of adefovir dipivoxil for preparing a pharmaceutical formulation for treating mammalian breast cancer wherein MMTV-like retroviruses are the etiological agents or human primary biliary cirrhosis wherein MMTV-like retroviruses are the etiological agents by administering from 10 mg to 100 mg adefovir dipivoxil.

In another aspect, the invention relates to the use of tenofovir disoproxil fumarate for preparing a pharmaceutical formulation for treating mammalian breast cancer wherein MMTV-like retroviruses are the etiological agents or human primary biliary cirrhosis wherein MMTV-like retroviruses are the etiological agents by administering from 10 mg to 1000 mg tenofovir disoproxil fumarate.

### Detailed Description of the Invention

To determine if tenofovir and adefovir might be effective against the MMTV-like virus implicated in PBC and breast cancer, the ability of their active metabolites, PMPApp and PMEApp, to directly inhibit MMTV RT activity was directly evaluated, along with 3 other HIV RT inhibitors (3.TCppp, FTCppp and AZTppp). MMTV particles were isolated and concentrated from MM5MT cells (ATCC) after induction with dexamethasone and insulin as previously described (Fine, D., et al., In Vitro 12:693-701, 1976). Lysed particles were assayed for RT activity using a standard filter-based, [³³P]dNTP incorporation assay (modifies from Wu, J., et al., J. Biol. Chem. 268:9980-9985, 1993). Inhibition of the RT activity was measured by adding the test compounds, the acyclic nucleoside diphosphophosphonates and cyclic nucleoside triphosphates, at various concentrations and 50% inhibitory concentrations (IC₅₀) were calculated for each compound. All compounds tested were active against the MMTV RT. The two acyclic analogs, PMPApp and PMEApp, had similar IC₅₀ values of approximately 4 µM while e 3TCppp and FTCppp had IC₅₀ values of 108µM and 44µM, respectively. The activity of AZTppp against the MMTV RT as previously observed was confirmed with an IC₅₀ value of 0.4µM.

Samples suspected of containing MMTV-like retroviral reverse transcriptase include natural or man-made materials such as living organisms; tissue or cell cultures; biological samples such as biological material samples (blood, serum, urine, cerebrospinal fluid, tears, sputum, saliva, tissue samples, and the like); laboratory samples; food, water, or air samples; bioproduct samples such as extracts of cells, particularly recombinant cells synthesizing a desired glycoprotein; and the like. Typically the sample will be suspected of containing an organism which produces MMTV-like reverse transcriptase, frequently a pathogenic organism such as an MMTV-like retrovirus. Samples can be contained in any medium including water and organic solvent\water mixtures. Samples include living organisms such as humans, and man made materials such as cell cultures.

The treating step comprises adding PMEApp or PMPApp to the sample or adding a precursor of PMEApp or PMPApp to the sample. The addition step comprises any way of administration as described herein.

If desired, the activity of the MMTV-like reverse transcriptase after application of the composition can be observed by any method including direct and indirect methods of detecting reverse transcriptase activity. Quantitative, qualitative, and semiquantitative methods of determining reverse transcriptase activity are all contemplated. Typically one of the screening methods described above are applied, however, any other method such as observation of the physiological properties of a living organism are also applicable.

### Pharmaceutical Formulations

The compounds for use in this invention are formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. All formulations will optionally contain excipients such as those set forth in the "Handbook of Pharmaceutical Excipients" (1986). Excipients include ascorbic acid and other antioxidants, chelating agents such as EDTA, carbohydrates such as dextran, hydroxyalkylcellulose, hydroxyalkylmethylcellulose, stearic acid and the like. The pH of the formulations ranges from about 3 to about 11, but is ordinarily about 7 to 10.

While it is possible for the active ingredients to be administered alone it may be preferable to present them as pharmaceutical formulations. The formulations, both for veterinary and for human use, for use in the invention comprise at least one active ingredient, as above defined, together with one or more acceptable carriers therefore and optionally other therapeutic ingredients. The carrier(s) must be "acceptable" in the sense of being compatible with the other ingredients of the formulation and physiologically innocuous to the recipient thereof.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet is made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom.

Pharmaceutical formulations for use according to the present invention comprise a combination according to the invention together with one or more pharmaceutically acceptable carriers or excipients and optionally other therapeutic agents. Pharmaceutical formulations containing the active ingredient may be in any form suitable for the intended way of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and whereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions for use in the invention contain the active materials in admixture with excipients suitable for the manufacture of aqueous suspensions. Such excipients include a suspending agent, such as sodium carboxymethylcellulose, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions for use in invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

The pharmaceutical compositions for use in the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butanediol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical composition can be prepared to provide easily measurable amounts for administration. For example, an aqueous solution intended for intravenous infusion may contain from about 3 to 500 µg of the active ingredient per milliliter of solution in order that infusion of a suitable volume at a rate of about 30 mL/hr can occur.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should be understood that in addition to the ingredients particularly mentioned above the formulation for use in this invention may include other agents conventional in the art having regard to the type of formulation in question, for example those suitable for oral administration may include flavoring agents.

Also disclosed are veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds for use in the invention are used to provide controlled release pharmaceutical formulations containing as active ingredient one or more compounds of the invention ("controlled release formulations") in which the release of the active ingredient are controlled and regulated to allow less frequency dosing or to improve the pharmacokinetic or toxicity profile of a given active ingredient.

Effective dose of active ingredient depends at least on the nature of the condition being treated, toxicity, whether the compound is being used prophylactically (lower doses) or against an active viral infection, the way of delivery, and the pharmaceutical formulation, and will be determined by the clinician using conventional dose escalation studies. It can be expected to be from 0.0001 to 100 mg/kg body weight per day. Typically, from 0.01 to 10 mg/kg body weight per day. More typically, from .01 to 5 mg/kg body weight per day. More typically, from .05 to 0.5 mg/kg body weight per day. For example, the daily candidate dose for an adult human of approximately 70 kg body weight will range from 1 mg to 1000 mg, preferably between 5 mg and 500 mg, and may take the form of single or multiple doses.

### Routes of Administration

One or more compounds for use in the invention (herein referred to as the active ingredients) are administered by any route appropriate to the condition to be treated. Suitable routes include oral, rectal, nasal, topical (including buccal and sublingual), vaginal and parenteral (including subcutaneous, intramuscular, intravenous, intradermal, intrathecal and epidural), and the like. It will be appreciated that the preferred route may vary with for example the condition of the recipient. An advantage of the compounds of this invention is that they are orally bioavailable and can be dosed orally.

### Combination Therapy

Compositions for use in the invention are also used in combination with other active ingredients. Such combinations are selected based on the condition to be treated, cross-reactivities of ingredients and pharmaco-properties of the combination. For example, the compositions for use in the invention may be combined with other antivirals such as protease inhibitors, nucleoside reverse transcriptase inhibitors, non-nucleoside reverse transcriptase inhibitors, integrase inhibitors or other immunomodulators, such as interferon.

It is possible to combine any compound for use in the invention with one or more other active ingredients in a unitary dosage form for simultaneous or sequential administration to an infected patient. The combination therapy may be administered as a simultaneous or sequential regimen. When administered sequentially, the combination may be administered in two or more administrations. Second and third active ingredients in the combination may have anti-MMTV activity. Exemplary active ingredients to be administered in combination with compounds for use in the invention are protease inhibitors, nucleoside reverse transcriptase inhibitors, non-nucleoside reverse transcriptase inhibitors, integrase inhibitors and cytokines, such as interferon.

The combination therapy may provide "synergy" and "synergistic", i.e. the effect achieved when the active ingredients used together is greater than the sum of the effects that results from using the compounds separately. A synergistic effect may be attained when the active ingredients are: (1) co-formulated and administered or delivered simultaneously in a combined formulation; (2) delivered by alternation or in parallel as separate formulations; or (3) by some other regimen. When delivered in alternation therapy, a synergistic effect may be attained when the compounds are administered or delivered sequentially, e.g. in separate tablets, pills or capsules, or by different injections in separate syringes. In general, during alternation therapy, an effective dosage of each active ingredient is administered sequentially, i.e. serially, whereas in combination therapy, effective dosages of two or more active ingredients are administered together. A synergistic anti-viral effect denotes an antiviral effect which is greater than the predicted purely additive effects of the individual compounds of the combination.

While specific examples have been provided, the above description is illustrative. The described embodiments are to be considered in all respects only as illustrative. The scope of the invention is, therefore, indicated by the appended claims.

## Claims

1. The use of adefovir or tenofovir for preparing a pharmaceutical formulation for treating mammalian breast cancer wherein MMTV-like retroviruses are the etiological agents or human primary biliary cirrhosis wherein MMTV-like retroviruses are the etiological agents by inhibiting the activity of MMTV-like retrovirus reverse transcriptase.

2. the use of adefovir or tenofovir for preparing a pharmaceutical formulation for treating human primary biliary cirrhosis wherein MMTV-like retroviruses are the etiological agents or mammalian breast cancer wherein MMTV-like retroviruses are the etiological agents by inhibiting the replication of MMTV-like retroviruses.

3. The use of adefovir or tenofovir according to claim 1 or 2 for treating mammalian breast cancer.

4. The use of adefovir or tenofovir according to claim 1 or 2 for treating human primary biliary cirrhosis.

5. The use of adefovir dipivoxil for preparing a pharmaceutical formulation for treating mammalian breast cancer wherein MMTV-like retroviruses are the etiological agents or human primary biliary cirrhosis wherein MMTV-like retroviruses are the etiological agents by administering from 10 mg to 100 mg adefovir dipivoxil.

6. The use of tenofovir disoproxil fumarate for preparing a pharmaceutical formulation for treating mammalian breast cancer wherein MMTV-like retroviruses are the etiological agents or human primary biliary cirrhosis wherein MMTV-like retroviruses are the etiological agents by administering from 10 mg to 1000 mg tenofovir disoproxil fumarate.

7. The use of adefovir dipivoxil or tenofovir disoproxil fumarate according to claim 5 or 6 for treating mammalian breast cancer.

8. The use of adefovir dipivoxil or tenofovir disoproxil fumarate according to claim 5 or 6 for treating human primary biliary cirrhosis.

9. The use of any one of claims 1 to 8 further comprising the use of a second therapeutic agent selected from other antivirals or immunomodulators.

## Patentansprüche

1. Verwendung von Adefovir oder Tenofovir zur Herstellung einer pharmazeutischen Formulierung zur Behandlung von Brustkrebs bei Säugern, wobei MMTV-artige Retroviren die ätiologischen Faktoren sind, oder von menschlicher primär biliärer Zirrhose, wobei MMTV-artige Retroviren die ätiologischen Faktoren sind, durch Inhibierung der Aktivität der reversen Transkriptase des MMTV-artigen Retrovirus.

2. Verwendung von Adefovir oder Tenofovir zur Herstellung einer pharmazeutischen Formulierung zur Behandlung menschlicher primär biliärer Zirrhose, wobei MMTV-artige Retroviren die ätiologischen Faktoren sind, oder von Brustkrebs bei Säugern, wobei MMTV-artige Retroviren die ätiologischen Faktoren sind, durch Inhibierung der Replikation der MMTV-artigen Retroviren.

3. Verwendung von Adefovir oder Tenofovir nach Anspruch 1 oder 2 zur Behandlung von Brustkrebs bei Säugern.

4. Verwendung von Adefovir oder Tenofovir nach Anspruch 1 oder 2 zur Behandlung menschlicher primär biliärer Zirrhose.

5. Verwendung von Adefovir-Dipivoxil zur Herstellung einer pharmazeutischen Formulierung zur Behandlung von Brustkrebs bei Säugern, wobei MMTV-artige Retroviren die ätiologischen Faktoren sind, oder von menschlicher primär biliärer Zirrhose, wobei MMTV-artige Retroviren die ätiologischen Faktoren sind, durch Verabreichung von 10 mg bis 100 mg Adefovir-Dipivoxil.

6. Verwendung von Tenofovir-Disoproxilfumarat zur Herstellung einer pharmazeutischen Formulierung zur Behandlung von Brustkrebs bei Säugern, wobei MMTV-artige Retroviren die ätiologischen Faktoren sind, oder von menschlicher primär biliärer Zirrhose, wobei MMTV-artige Retroviren die ätiologischen Faktoren sind, durch Verabreichung von 10 mg bis 1000 mg Tenofovir-Disoproxilfumarat.

7. Verwendung von Adefovir-Dipivoxil oder Tenofovir-Disoproxilfumarat nach Anspruch 5 oder 6 zur Behandlung von Brustkrebs bei Säugern.

8. Verwendung von Adefovir-Dipivoxil oder Tenofovir-Disoproxilfumarat nach Anspruch 5 oder 6 zur Behandlung menschlicher primär biliärer Zirrhose.

9. Verwendung nach einem der Ansprüche 1 bis 8, weiterhin umfassend die Verwendung eines zweiten therapeutischen Mittels, ausgewählt unter anderen antiviralen Mitteln oder Immunmodulatoren.

## Revendications

1. Utilisation d'adéfovir ou de ténofovir pour préparer une formulation pharmaceutique conçue pour traiter, chez un mammifère, un cancer du sein dont les agents étiologiques sont des rétrovirus apparentés au virus MMTV, ou chez un humain, une cirrhose biliaire primitive dont les agents étiologiques sont des rétrovirus apparentés au virus MMTV, par inhibition de l'activité de la transcriptase inverse des rétrovirus apparentés au virus MMTV.

2. Utilisation d'adéfovir ou de ténofovir pour préparer une formulation pharmaceutique conçue pour traiter, chez un humain, une cirrhose biliaire primitive dont les agents étiologiques sont des rétrovirus apparentés au virus MMTV, ou chez un mammifère, un cancer du sein dont les agents étiologiques sont des rétrovirus apparentés au virus MMTV, par inhibition de la réplication des rétrovirus apparentés au virus MMTV.

3. Utilisation d'adéfovir ou de ténofovir, conforme à la revendication 1 ou 2, pour traiter un cancer du sein chez un mammifère.

4. Utilisation d'adéfovir ou de ténofovir, conforme à la revendication 1 ou 2, pour traiter une cirrhose biliaire primitive chez un humain.

5. Utilisation d'adéfovir dipivoxil pour préparer une formulation pharmaceutique conçue pour traiter, chez un mammifère, un cancer du sein dont les agents étiologiques sont des rétrovirus apparentés au virus MMTV, ou chez un humain, une cirrhose biliaire primitive dont les agents étiologiques sont des rétrovirus apparentés au virus MMTV, par administration de 10 mg à 100 mg d'adéfovir dipivoxil.

6. Utilisation de fumarate de ténofovir disoproxil pour préparer une formulation pharmaceutique conçue pour traiter, chez un mammifère, un cancer du sein dont les agents étiologiques sont des rétrovirus apparentés au virus MMTV, ou chez un humain, une cirrhose biliaire primitive dont les agents étiologiques sont des rétrovirus apparentés au virus MMTV, par administration de 10 mg à 1000 mg de fumarate de ténofovir disoproxil.

7. Utilisation d'adéfovir dipivoxil ou de fumarate de ténofovir disoproxil, conforme à la revendication 5 ou 6, pour traiter un cancer du sein chez un mammifère.

8. Utilisation d'adéfovir dipivoxil ou de fumarate de ténofovir disoproxil, conforme à la revendication 5 ou 6, pour traiter une cirrhose biliaire primitive chez un humain.

9. Utilisation conforme à l'une des revendications 1 à 8, qui comprend en outre l'utilisation d'un deuxième agent thérapeutique choisi parmi d'autres agents anti-viraux ou immuno-modulateurs.
